# EUROPEAN PATENT SPECIFICATION

(11) **EP 1 827 602 B1**
(45) Date of publication and mention of the grant of the patent: **09.03.2011**
(21) Application number: 04822466.1
(22) Date of filing: 26.11.2004
(51) Int. Cl.: C12N 15/11, A61K 31/711, A61P 27/02

(54) **MODULATING RETINAL PIGMENTED EPITHELIUM PERMEAION BY INHIBITING VEGFR-1**
MODULIERUNG DER RETINALEN PIGMENTIERTEN EPITHEL-PERMEATION DURCH HEMMUNG VON VEGFR-1
MODULATION DE LA PERMEATION DE L'EPITHELIUM PIGMENTAIRE RETINIEN PAR INHIBITION DU VEGFR-1

(43) Date of publication of application: 05.09.2007
(73) Proprietor: Novagali Pharma S.A., 91000 Evry (FR); Institut National de la Santé et de la Recherche Médicale (INSERM), 75654 Paris Cedex 13 (FR)
(72) Inventor: MIYAMOTO, Noriko, Hyogo 666-0137 (JP); NORMAND, Nadia, F-92100 Boulogne (FR); PLOUET, Jean, F-75014 Paris (FR); BEHAR-COHEN, Francine, F-75016 Paris (FR)
(74) Representative: Icosa
(86) International application number: PCT/IB2004/004107
(87) International publication number: WO 2006/056823

(56) References cited:
- EP-A- 0 882 799
- WO-A-98/13071
- WO-A-03/012105
- WO-A-03/062788
- WO-A-03/092669
- WO-A-2005/000223
- US-A1- 2002 009 750
- US-A1- 2003 105 036
- US-B1- 6 245 512
- OHNO-MATSUI KYOKO ET AL: "Vascular endothelial growth factor upregulates pigment epithelium-derived factor expression via VEGFR-1 in human retinal pigment epithelial cells." BIOCHEMICAL AND BIOPHYSICAL RESEARCH COMMUNICATIONS. 11 APR 2003, vol. 303, no. 3, 11 April 2003 (2003-04-11), pages 962-967, XP002334147 ISSN: 0006-291X
- DATABASE MEDLINE [Online] US NATIONAL LIBRARY OF MEDICINE (NLM), BETHESDA, MD, US; 10 October 2002 (2002-10-10), LEI HETIAN ET AL: "[Inhibition of tumor growth by a peptide fusion protein binding to vascular endothelial growth factor receptor Flt-1]" XP002334149 Database accession no. NLM12509940 & ZHONGHUA YI XUE ZA ZHI. 10 OCT 2002, vol. 82, no. 19, 10 October 2002 (2002-10-10), pages 1342-1345, ISSN: 0376-2491
- UEDA YASUJI ET AL: "A novel low molecular weight antagonist of vascular endothelial growth factor receptor binding: VGA1155." MOLECULAR CANCER THERAPEUTICS. NOV 2003, vol. 2, no. 11, November 2003 (2003-11), pages 1105-1111, XP002334148 ISSN: 1535-7163
- "Inhibition of retinal neovascularization by intraocular gene delivery of soluble VEGF receptors" MOLECULAR THERAPY, ACADEMIC PRESS, SAN DIEGO, CA,, US, vol. 9, May 2004 (2004-05), page 198, XP004634889 ISSN: 1525-0016
- MALIK A K HANS-PETER GERBER: "Targeting VEGF ligands and receptors in cancer" TARGETS, ELSEVIER, vol. 2, no. 2, 1 April 2003 (2003-04-01), pages 48-57, XP004886645 ISSN: 1477-3627
- AUTIERO M ET AL: "Placental growth factor and its receptor, vascular endothelial growth factor receptor-1: novel targets for stimulation of ischemic tissue revascularization and inhibition of angiogenic and inflammatory disorders." JOURNAL OF THROMBOSIS AND HAEMOSTASIS : JTH. JUL 2003, vol. 1, no. 7, July 2003 (2003-07), pages 1356-1370, XP009050026 ISSN: 1538-7933

## Description

The present invention provide means for modulating the permeability of the retinal pigmented epithelial layer, for treating or preventing various diseases associated with permeability disorders of the retinal pigmented epithelial layer (RPE).

### BACKGROUND OF INVENTION

VEGF-A (vascular endothelial growth factor) is an homodimeric protein that contains one of the five VEGF isoforms: 121, 145, 165, 189 or 206 amino acids (VEGF-121, -145, -165, -189, or -206) (Ferrara et al. Nat Med. 2003 Jun;9(6):669-76. Review; Gerber et al., J Biol Chem. 1997 Sep 19;272(38):23659-67). VEGF receptors have been identified in vascular endothelial cells and in pericytes (Witmer et al., Prog Retin Eye Res. 2003 Jan;22(1):1-29. Review). Two high affinity receptors for VEGF, named VEGFR-1 or Flt-1 and VEGFR-2 or KDR (Flk-1 in mouse) have been characterised. They are both transmembrane proteins with cytoplasmic tyrosine kinase domains, and are expressed on endothelial cells. The two receptors appear to mediate different biological effects of VEGF. Mice lacking the complete Flt-1 gene display an increased number of endothelial progenitors and vascular disorganization and die in utero at embryonic day 9 (Fong et al. Nature 1995, 376:66-70). Mice in which the f1k-1/KDR gene has been inactivated also die at embryonic day 9. They are deficient in vasculogenesis and also lack blood island formation (Shalaby et al. Nature 1995, 376:62-66).

Placental growth factor (P1GF) is an homodimeric protein that shares substantial structural similarity with VEGF-A. Three P1GF isoforms have been described (P1GF-1, -2 and-3). These isoforms do not interact with VEGFR-2 but do bind to VEGFR-1 (Christinger et al, J Biol Chem. 2004 Mar 12;279(11):10382-8. Epub 2003 Dec 18.).

It is generally accepted that activation of VEGFR-2 induces angiogenesis and endothelial cell proliferation (Millauer et al., Nature. 1994 Feb 10;367(6463):576-9 ; Jonca et al., J Biol Chem. 1997 Sep 26;272(39):24203-9) while activation of VEGFR-1 mostly induces migration of endothelial cells (Barleon et al., Blood. 1996 Apr 15;87(8):3336-43) and monocytes (Barleon et al., 1996 ; Autiero et al., J Thromb Haemost. 2003 Jul;1(7):1356-70. Review).

It has been demonstrated that VEGF plays an important role in neovascular diseases in animal models and in humans. VEGF enhances pathological angiogenesis in synergy with other cytokines, and induces vascular permeability (Witmer et al., 2003; Caldwell et al, Diabetes Metab Res Rev. 2003 Nov-Dec;19(6):442-55. Review). In vivo, VEGF potently increases microvascular permeability of post capillary venules and capillaries, and can rapidly induce fenestrations in continuous endothelia when injected in skin or muscle. This effect on endothelial permeability is probably involved in the pathogenesis of malignant ascites, ovarian hyperstimulation syndrome and oedema around brain tumours, bullous skin diseases, and psoriasis.

The role of VEGFR-1 in angiogenesis was studied by several groups. Marchand et al. demonstrated that antisenses sequence directed against Flt-1 (SEQ ID N°2: 5'-AAGCAGACACCAGAGCAG-3' and SEQ ID N°3: 5'-CCCTGAGCCATATCCTGT-3') reduced by ~80% the VEGF-induced formation of new blood vessels in mouse testis (Marchand et al., Am J Physiol Heart Circ Physiol. 2002 Jan;282(1):H194-204).

In vitro, a chimeric protein containing the soluble form of VEGFR-1 receptor (sFlt-1) suppressed the angiogenic growth capacity of endothelial cells from patients with rheumatoid arthritis (Sekimoto et al., J Rheumatol. 2002 Feb;29(2):240-5). Treatment with sFlt-1, either transfected or administered by itself, proved its therapeutic efficacy antagonizing VEGF in various models of ovarian tumors (Mahasreshti et al., Clin Cancer Res. 2001 Jul;7(7):2057-66, Hasumi et al., Cancer Res. 2002 Apr 1;62(7):2019-23), thyroid carcinoma (Ye et al., Endocrinology. 2004 Feb;145(2):817-22), lung metastasis (Yoshimura et al., J Urol. 2004 Jun;171(6 Pt 1):2467-70). Also monoclonal antibodies directed against VEGFR-1 inhibited the growth of C6 glioma in a mouse xenograft (Stefanik et al., J Neurooncol. 2001 Nov;55(2):91-100). Gene transfer of VEGFR-1 mutants successfully decreased vascular density and tumor cell proliferation in vivo (Heidenreich et al., Int J Cancer. 2004 Sep 1;111(3):348-57). In addition, An et al. (Int J Cancer. 2004 Aug 20;111 (2):165-73) recently demonstrated in vivo successful tumor growth and metastasis inhibition by administration of an enzyme-bound VEGFR-1 antagonizing peptide.

The potential of this approach is also reflected by the numerous applied patents.

For example, US 6,383,486 describes novel chimeric VEGF receptor proteins comprising amino acid sequences derived from the vascular endothelial growth factor (VEGF) receptor flt-1 which antagonize the endothelial cell proliferative and angiogenic activity allowing utilizing such proteins for the treatment of conditions associated with undesired vascularization.

US 6,057,428 describes the modification of the kinase domain region of VEGFR-1 such that the binding affinity to native VEGF is modified.

US 6,375,929 describes the use of gene therapy for expressing soluble VEGFR-1 form for inhibiting angiogenesis associated with solid tumor growth, tumor metastasis, inflammation, psoriasis, rheumatoid arthritis, hemangiomas, diabetic retinopathy, angiofibromas, and macular degeneration.

US 6,245,512 describes a method for regulating VEGFR-1 concentration by regulating the activity of the receptor promoter.

US 6,365,157 and 6,448,077 describe monoclonal, chimeric and humanized antibodies that specifically bind to the VEGFR-1 receptor and neutralize activation of the receptor, providing a method for reducing tumor growth in a mammal.

US 6,114,320 discloses a method using a selective PKC inhibitor for inhibiting VEGF stimulated endothelial cell growth, such as associated with macular degeneration, and VEGF stimulated capillary permeability, such as associated with macular edema.

US 6,617,160 describes a monoclonal antibody which immunologically reacts with human VEGF receptor Flt-1 and inhibits its binding, providing means for the diagnosis or treatment of diseases such as proliferation or metastasis of solid tumors, arthritis in rheumatoid, arthritis, diabetic retinopathy, retinopathy of prematurity and psoriasis.

Disclosed and claimed in US 6,696,252 are antisense sequences capable of modulate indistinctinly VEGFR-1 and VEGFR-2. Disclosed and claimed in US 6,710,174 are antisense sequences capable of regulating VEGFR-1.

Despite all the above mentioned studies, there is still significant uncertainty concerning the role of individual VEGF receptors for other biological effects than mitogenesis, such as permeability.

Stacker et al. show that a VEGF mutant chimeric protein that is unable to trigger VEGFR-2 activation can cause vascular permeability to an extent that is similar to wild type VEGF, concluding that Flt-1 could be responsible for the increased VEGF-induced vascular permeability (Stacker et al., J Biol Chem. 1999 Dec 3;274(49):34884-92). Gille et al. reported that while a selective VEGFR-2 agonist induced comparable vascular permeability as the wild type VEGF, the VEGFR-1 selective VEGF caused essentially no leakage even at high concentrations in the rat cornea. Also in dye extravasation tests a known VEGFR-1 agonist (P1GF) failed to induce any vascular permeability (Gille et al., J Biol Chem. 2001 Feb 2;276(5):3222-30).

A recent report describes the increased vascular permeability induced by a protein originated from snake venom which has VEGFR-1 agonist activity but binds to VEGFR-2 weakly (Takahashi et al., J Biol Chem. 2004 Oct 29;279 (44):46304-14).

Angiogenesis plays also a crucial role in disorders responsible for most blind registration in the Western world. VEGF has been shown particularly to be associated with numerous ocular pathologic conditions associated with angiogenesis, including the pathogenesis of diabetic retinopathy (DR), retinopathy of prematurity (ROP), age-related macular degeneration (ARMD) and ocular neovascularization (ON). VEGF is produced by vascular retinal endothelial cells and pericytes, retinal pigment epithelial cells (RPE), astocytes and retinal Müller glial cells (RMG) (Simorre-Pinatel et al, Invest Ophthalmol Vis Sci. 1994 Aug;35(9):3393-400 ; Adamis et al., Biochem Biophys Res Commun. 1993 Jun 15;193(2):631-8 ; Behzadian et al., Glia. 1998 Oct;24(2):216-25).

VEGF receptors have been identified in RPE cells, inner retina and in synaptic complexes of cone-photoreceptors (Witmer et al., 2003). VEGFR-1 has been identified as the predominant VEGF receptor in pericyte microvessels of normal human and monkey adult retinas (Witmer et al., 2003 ; Witmer et al., Invest Ophthalmol Vis Sci. 2002 Mar;43(3):849-57). It is also present in the inner retina (Witmer et al., 2003) and in human RPE cells (Ohno-Matsui et al., Biochem Biophys Res Commun. 2003 Apr 11;303(3):962-7).

In ophthalmology, inhibition of angiogenesis was also proved to be of great value. Injection or transfection with sFlt-1 in the anterior chamber inhibited corneal and choroidal angiogenesis in animal models of neovascularization (Lai et al., Hum Gene Ther. 2001 Jul 1;12(10):1299-310, Exp Eye Res. 2002 Dec;75(6):625-34). Antibodies to the receptor demonstrated as well therapeutic potential suppressing neovascularization in ischemic retina (Luttun et al., Nat Med. 2002 Aug;8(8):831-40) and Rota et al. showed in the rat model of ROP impressive reduction in retinal neovascularization following intravitreal injection of an adenoviral vector expressing sFlt-1 (Rota et al., J Gene Med. 2004 Sep;6(9):992-1002).

The role of VEGFR-1 on the retinal pigmented epithelial barrier has however never been elucidated. The inventors have discovered the role of VEGFR-1 on the retinal permeability.

Consequently the instant invention concerns the potential applications of agonists or antagonists of VEGFR-1 in the ophthalmic domain for regulating the external hemato-retinal barrier.

### DESCRIPTION OF THE INVENTION

The invention is as defined in the claims.

The present invention relates to Antagonist des vaskulären endothelialen Wachstumsfaktor-Rezeptor-1 (VEGFR-I) zur Verwendung in der Vorbeugung oder der Behandlung eines Patienten leidend an einer Augenerkrankung, wobei Erkrankungen der retinalen Pigmentepithel-(RPE)-Permeation involviert sind, wobei der Antagonist ein Antisinn-Oligonukleotid der mRNA kodierend für VEGFR-I ist, wobei das Antisinn-Oligonukleotid die Sequenz SEQ ID NO: 1 hat, und wobei die Augenerkrankung ausgewählt ist aus der Gruppe umfassend Makulaödem, zentrale seröse Chorioretinopathie, Epitheliophathie und altersabhängige Makuladegeneration (ARMD).

Cellular permeability can be assessed by transepithelial electrical resistance (TER) and tritiated inulin flux across RPE monolayers (see point 1.1 of the examples).

The expression "antagonist of VRGFR-1" refers in the present description to any compound which, when it binds to VEGFR-1, reduces RPE cell permeability by at least 25%, preferably 30, 40, 50, 60, 70 or 80%. As described above, cellular permeability can be assessed by TER and tritiated inulin flux across RPE monolayers.

An antagonist of VEGFR-1 can be selected from the group comprising blocking antibodies such as MF1 antifltlmAb, peptides such as DHFR-F56/F90 (Lei.H et al. Zhonghua Yi Xue Za Zhi. 2002 Oct 10;82(19):1342-5), oligoaptamer, ribozymes, siRNA, antisense oligonucleotides, or compounds such as immunomodulator compounds, for example FTY720 (Sanchez et al., J Biol Chem. 2003 Nov 21;278(47):47281-90), 5-[N-methyl-N-(4-octadecyloxyphenyl)-acetyl] amino-2-methylthiobenzoic acid (Ueda et al., Mol Cancer Ther. 2003 Nov;2(11):1105-11) and acetyl salicilate.

The antagonist is an antisense oligonucleotide of the mRNA encoding VEGFR-1, said antisense oligonucleotide having the sequence SEQ ID N°1.

In the context of this description, the term "oligonucleotide" refers to an oligomer or polymer of ribonucleic acid (RNA) or deoxyribonucleic acid (DNA) or mimetics thereof. This term includes oligonucleotides composed of naturally-occurring nucleobases, sugars and covalent internucleoside (backbone) linkages as well as oligonucleotides having non-naturally-occurring portions which function similarly. Such modified or substituted oligonucleotides are often preferred over native forms because of desirable properties such as, for example, enhanced cellular uptake, enhanced affinity for nucleic acid target and increased stability in the presence of nucleases.

The present application describes other oligomeric antisense compounds, including but not limited to oligonucleotide mimetics such as are described below. The antisense compounds preferably comprise from about 8 to about 50 nucleobases (i.e. from about 8 to about 50 linked nucleosides). Particularly preferred antisense compounds are antisense oligonucleotides, even more preferably those comprising from about 12 to about 30 nucleobases. Antisense compounds include ribozymes, external guide sequence (EGS) oligonucleotides (oligozymes), and other short catalytic RNAs or catalytic oligonucleotides which hybridize to the target nucleic acid and modulate its expression.

As known in the art, a nucleoside is a base-sugar combination. The base portion of the nucleoside is normally a heterocyclic base. The two most common classes of such heterocyclic bases are the purines and the pyrimidines. Nucleotides are nucleosides that further include a phosphate group covalently linked to the sugar portion of the nucleoside. For those nucleosides that include a pentofuranosyl sugar, the phosphate group can be linked to the 2', 3' or 5' hydroxyl moiety of the sugar. In forming oligonucleotides, the phosphate groups covalently link adjacent nucleosides to one another to form a linear polymeric compound. In turn the respective ends of this linear polymeric structure can be further joined to form a circular structure, however, open linear structures are generally preferred. Within the oligonucleotide structure, the phosphate groups are commonly referred to as forming the internucleoside backbone of the oligonucleotide. The normal linkage or backbone of RNA and DNA is a 3' to 5' phosphodiester linkage.

Specific examples of preferred antisense compounds include oligonucleotides containing modified backbones or non-natural internucleoside linkages. As defined in this specification, oligonucleotides having modified backbones include those that retain a phosphorus atom in the backbone and those that do not have a phosphorus atom in the backbone. For the purposes of this specification, and as sometimes referenced in the art, modified oligonucleotides that do not have a phosphorus atom in their internucleoside backbone can also be considered to be oligonucleosides.

Preferred modified oligonucleotide backbones include, for example, phosphorothioates, chiral phosphorothioates, phosphorodithioates, phosphotriesters, aminoalkylphosphotriesters, methyl and other alkyl phosphonates including 3'-alkylene phosphonates, 5'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, selenophosphates and borano-phosphates having normal 3'-5' linkages, 2'-5' linked analogs of these, and those having inverted polarity wherein one or more internucleotide linkages is a 3' to 3', 5' to 5' or 2'to 2' linkage. Preferred oligonucleotides having inverted polarity comprise a single 3' to 3' linkage at the 3'-most internucleotide linkage i.e. a single inverted nucleoside residue which may be abasic (the nucleobase is missing or has a hydroxyl group in place thereof). Various salts, mixed salts and free acid forms are also included.

Representative United States patents that teach the preparation of the above phosphorus-containing linkages include, but are not limited to, US. 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; 5,194,599; 5,565,555; 5,527,899; 5,721,218; 5,672,697 and 5,625,050, certain of which are commonly owned with this application.

Preferred modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and CH.sub.2 component parts.

Representative United States patents that teach the preparation of the above oligonucleosides include, but are not limited to, US 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; 5,792,608; 5,646,269 and 5,677,439, certain of which are commonly owned with this application. See US 6,710,174 for further information concerning replacement of sugar, internucleoside linkage or nucleobase with respectively novel groups or nucleobases, or concerning oligonucleotide conjugates.

The antisense compounds may be conveniently and routinely made through the well-known technique of solid phase synthesis. Equipment for such synthesis is sold by several vendors including, for example, Applied Biosystems (Foster City, Calif.). Any other means for such synthesis known in the art may additionally or alternatively be employed. It is well known to use similar techniques to prepare oligonucleotides such as the phosphorothioates and alkylated derivatives.

The antisense compounds are synthesized in vitro and may include antisense compositions of biological origin, or genetic vector constructs designed to direct the in vivo synthesis of antisense molecules (see for example Current Protocols in Molecular Biology, Volumes I, II, and III, 1997 (F. M. Ausubel ed.); Sambrook et al., 1989, Molecular Cloning: A Laboratory Manual, Second Edition, Cold Spring Harbor Laboratory Press, Cold Spring Harbor, N.Y.; DNA Cloning: A Practical Approach, Volumes I and II, 1985 (D. N. Glover ed.); Oligonucleotide Synthesis, 1984 (M. L. Gait ed.); Nucleic Acid Hybridization, 1985, (Hames and Higgins); Transcription and Translation, 1984 (Hames and Higgins eds.); Animal Cell Culture, 1986 (R. I. Freshney ed.); Immobilized Cells and Enzymes, 1986 (IRL Press); Perbal, 1984, A Practical Guide to Molecular Cloning; the series, Methods in Enzymology (Academic Press, Inc.); Gene Transfer Vectors for Mammalian Cells, 1987 (J. H. Miller and M. P. Calos eds., Cold Spring Harbor Laboratory); and Methods in Enzymology Vol. 154 and Vol. 155 (Wu and Grossman, and Wu, eds., respectively) for conventional techniques in molecular biology, microbiology, and recombinant DNA). The compounds may also be admixed, encapsulated, conjugated or otherwise associated with other molecules, molecule structures or mixtures of compounds, as for example, liposomes, receptor targeted molecules, oral, rectal, topical or other formulations, for assisting in uptake, distribution and/or absorption.

Representative United States patents that teach the preparation of such uptake, distribution and/or absorption assisting formulations include, but are not limited to, US 5,108,921; 5,354,844; 5,416,016; 5,459,127; 5,521,291; 5,543,158; 5,547,932; 5,583,020; 5,591,721; 4,426,330; 4,534,899; 5,013,556; 5,108,921; 5,213,804; 5,227,170; 5,264,221; 5,356,633; 5,395,619; 5,416,016; 5,417,978; 5,462,854; 5,469,854; 5,512,295; 5,527,528; 5,534,259; 5,543,152; 5,556,948; 5,580,575; and 5,595,756.

The antisense compounds encompass any pharmaceutically acceptable salts, esters, or salts of such esters, or any other compound which, upon administration to an animal including a human, is capable of providing (directly or indirectly) the biologically active metabolite or residue thereof. Accordingly, for example, the disclosure is also drawn to prodrugs and pharmaceutically acceptable salts of the compounds of the invention, pharmaceutically acceptable salts of such prodrugs, and other bioequivalents.

The term "prodrug" indicates a therapeutic agent that is prepared in an inactive form that is converted to an active form (i.e., drug) within the body or cells thereof by the action of endogenous enzymes or other chemicals and/or conditions. In particular, prodrug versions of the oligonucleotides of the invention are prepared as SATE [(S-acetyl-2-thioethyl) phosphate] derivatives according to the methods disclosed in WO 93/24510 to Gosselin et al., published Dec. 9, 1993 or in WO 94/26764 and US 5,770,713 to Imbach et al.

The term "pharmaceutically acceptable salts" refers to physiologically and pharmaceutically acceptable salts of the compounds of the invention: i.e., salts that retain the desired biological activity of the parent compound and do not impart undesired toxicological effects thereto, and are well known by the man skilled in the art.

By percentage of identity between two nucleic acid or amino acid sequences in the present invention, it is meant a percentage of identical nucleotides or amino acid residues between the two sequences to compare, obtained after the best alignment ; this percentage is purely statistical, and the differences between the two sequences are randomly distributed and all along their length. The best alignment or optimal alignment is the alignment corresponding to the highest percentage of identity between the two sequences to compare, which is calculated such as herein after. The sequence comparisons between two nucleic acid or amino acid sequences are usually performed by comparing these sequences after their optimal alignment, said comparison being performed for one segment or for one "comparison window", to identify and compare local regions of sequence similarity. The optimal alignment of sequences for the comparison can be performed manually or by means of the algorithm of local homology of Smith and Waterman (1981) (Ad. App. Math. 2:482), by means of the algorithm of local homology of Neddleman and Wunsch (1970) (J. Mol. Biol. 48:443), by means of the similarity research method of Pearson and Lipman (1988) (Proc. Natl. Acad. Sci. USA 85:2444), by means of computer softwares using these algorithms (GAP, BESTFIT, FASTA and TFASTA in the Wisconsin Genetics Software Package, Genetics Computer Group, 575 Science Dr., Madison, WI).

The percentage of identity between two nucleic acid or amino acid sequences is determined by comparing these two aligned sequences in an optimal manner with a "comparison window" in which the region of the nucleic acid or amino acid sequence to compare may comprise additions or deletions with regard the sequence of reference for an optimal alignment between these two sequences. The percentage of identity is calculated by determining the number of positions for which the nucleotide or the amino acid residue is identical between the two sequences, by dividing this number of identical positions by the total number of positions in the "comparison window" and by multiplying the result obtained by 100, to obtain the percentage of identity between these two sequences.

According to the instant invention, the subject, also named herein "patient", is a mammal, preferably a human being.

The instant invention also provides a pharmaceutical composition comprising an antisense oligonucleotide of the mRNA encoding VEGFR-I, said antisense oligonucleotide having the sequence SEQ ID N°1, together with a pharmaceutically acceptable carrier.

The pharmaceutical composition can be used for preventing or treating a subject suffering from an ocular disease wherein disorders of the RPE permeation are involved.

In a specific embodiment of the invention, the ocular disease wherein disorders of the RPE permeation are involved is one listed above.

The invention further relates to a pharmaceutical composition containing a first compound which is an antagonist of VEGFR-1 having the sequence SEQ ID N°1 and a second active compound as defined below, a combined preparation for simultaneous, separate or sequential use to treat an ocular disease.

In this case, said antagonist of VEGFR-1 modulates the intraocular penetration of the second active compound systemically administered.

In a specific embodiment, the ocular disease is selected from the group comprising macular edema of any origin such a those associated or not to serous detachment of RPE cells, central serous chorioretinopathy, epitheliopathy of any origin, age related macular degeneration (ARMD) such as ARMD with leakage from the choriocapillaries into the subretinal space, infections, intraocular inflammations, retinal dystrophy of any origin, diabetic retinopathy, ischemic retinopathy, optic neuritis, and any ocular diseases associated to neo vascular and/or fibroglial proliferations.

In a preferred embodiment of the invention, the ocular disease is an ocular disease wherein disorders of the RPE permeation are involved.

According to the instant invention, the second active compound is selected in the group comprising anti angiogenic compounds, non steroidal anti inflammatory, cortico and mineralo corticoids, anti-oxidants, lipids, neurotrophic factors (ciliary neurotrophic factor or CNTF, glial cell line-derived neurotrophic factor or GDNF, brain-derived neurotrophic factor or BDNF, fibroblast growth factor or FGF), COX inhibitors and antibiotics.

The a combined preparation can be administered simultaneously, separately or sequentially for preventing or treating a subject suffering from an ocular disease, the first compound modulating the intraocular penetration of the second active compound systemically administered.

In a specific embodiment of the invention, the ocular disease is selected from the group comprising macular edema of any origin such as those associated or not to serous detachment of RPE cells, central serous chorioretinopathy, epitheliopathy of any origin, age related macular degeneration (ARMD) such as ARMD with leakage from the choriocapillaries into the subretinal space, infections, intraocular inflammations, retinal dystrophy of any origin, diabetic retinopathy, ischemic retinopathy, optic neuritis, and any ocular diseases associated to neo vascular and/or fibroglial proliferations.

In a further specific embodiment of the invention, the second active compound is selected from the group comprising anti angiogenic compounds, non steroidal anti inflammatory, cortico and mineralo corticoids, anti-oxiants, lipids, neurotrophic factors (ciliary neurotrophic factor or CNTF, glial cell line-derived neurotrophic factor or GDNF, brain-derived neurotrophic factor or BDNF, fibroblast growth factor or FGF), COX inhibitors and antibiotics.

The ocular disease may be of any type as listed above.

The pharmaceutical composition of the invention comprises molecules as described above and a pharmaceutically acceptable carrier or excipient (both terms can be used interchangeably) to treat diseases as indicated above. Suitable carriers or excipients known to the skilled man are saline, Ringer's solution, dextrose solution, Hank's solution, fixed oils, ethyl oleate, 5% dextrose in saline, substances that enhance isotonicity and chemical stability, buffers and preservatives. Other suitable carriers include any carrier that does not itself induce the production of antibodies harmful to the individual receiving the composition such as micelles, emulsions, proteins, polysaccharides, polylactic acids, polyglycolic acids, polymeric amino acids and amino acid copolymers. Further details on techniques for formulation and administration may be found in the latest edition of Remington's Pharmaceutical Sciences (Maack Publishing Co., Easton, Pa.).

The medicament may be administered by any suitable method within the knowledge of the skilled man. One route of administration is parenterally. In parenteral administration, the medicament of this invention will be formulated in a unit dosage injectable form such as a solution, suspension or emulsion, in association with the pharmaceutically acceptable excipients as defined above. The preferred route of administration is ocularly, for example by transcleral delivery of bioreactive protein to the choroid and retina (Ambati et al. (2000) Investigative Ophthalmology and Visual Science, 41, 1186). The formulation of topical ophthalmic preparations, including ophthalmic solutions, suspensions and ointments is well known to those skilled in the art (see Remington's Pharmaceutical Sciences, 18th Edition, Chapter 86, pages 1581-1592 Mack Publishing Company, 1990). Other modes of administration are available, including intracameral injections (which may be made directly into the anterior chamber or directly into the vitreous chamber), subconjunctival injections and retrobulbar injections, and methods and means for producing ophthalmic preparations suitable for such modes of administration are also well known.

The term "extraocular" refers to the ocular surface and the (external) space between the eyeball and the eyelid. Examples of extraocular regions include the eyelid fornix or cul-de-sac, the conjunctival surface and the corneal surface. This location is external to all ocular tissues and an invasive procedure is not required to access this region. Examples of extraocular systems include inserts and "topically" applied drops, gels or ointments which may be used to deliver therapeutic material to these regions. Extraocular devices are generally easily removable, even by the patient.

The following patents disclose extraocular systems which are used to administer drugs to the extraocular regions. Higuchi et al. discloses in U.S. Pat. Nos. 3,981,303, 3,986,510 and 3,995,635, a biodegradable ocular insert which contains a drug. The insert can be made in different shapes for retention in the cul-de-sac of the eyeball, the extraocular space between the eyeball and the eyelid. Several common biocompatible polymers are disclosed as suitable for use in fabricating this device. These polymers include zinc alginate, poly (lactic acid), poly (vinyl alcohol), poly (anhydrides) and poly (glycolic acid). The patents also describe membrane coated devices with reduced permeation to the drug and hollow chambers holding the drug formulation.

Theeuwes, US 4,217,898, discloses microporous reservoirs which are used for controlled drug delivery. These devices are placed extraocularly in the ocular cul-de-sac. Among the polymer systems of interest include poly (vinylchloride)-co-poly (vinyl acetate) copolymers. Kaufman discloses in US 4,865,846 and 4,882,150 an ophthalmic drug delivery system which contains at least one bio-erodible material or ointment carrier for the conjunctival sac. The patent discloses polymer systems, such as, poly (lactide), poly (glycolide), poly (vinyl alcohol) and cross linked collagen, as suitable delivery systems.

It is also advantageous that a topically applied ophthalmic formulation includes an agent to promote the penetration or transport of the therapeutic agent into the eye. Such agents are known in the art. For example, Ke et al., US 5,221,696 discloses the use of materials to enhance the penetration of ophthalmic preparations through the cornea.

Intraocular systems are those systems which are suitable for use in any tissue compartment within, between or around the tissue layers of the eye itself. These locations include subconjunctival (under the ocular mucous membrane adjacent to the eyeball), orbital (behind the eyeball), and intracameral (within the chambers of the eyeball itself). In contrast to extraocular systems, an invasive procedure consisting of injection or implantation is required to access these regions.

The following patents disclose intraocular devices. Wong, US 4,853,224, discloses microencapsulated drugs for introduction into the chamber of the eye. Polymers which are used in this system include polyesters and polyethers. Lee, US 4,863,457, discloses a biodegradable device which is surgically implanted intraocularly for the sustained release of therapeutic agents. The device is designed for surgical implantation under the conjunctiva (mucous membrane of the eyeball). Krezancaki, US 4,188,373, discloses a pharmaceutical vehicle which gels at human body temperature. This vehicle is an aqueous suspension of the drug and gums or cellulose derived synthetic derivatives. Haslam et al. discloses in US 4,474,751 and 4,474,752 a polymer-drug system which is liquid at room temperature and gels at body temperature. Suitable polymers used in this system include polyoxyethylene and polyoxy propylene. Davis et al. disclose in US 5,384,333 a biodegradable injectable drug delivery polymer which provides long term drug release. The drug composition is made up of a pharmaceutically active agent in a biodegradable polymer matrix, where the polymer matrix is a solid at temperatures in the range 20.degree. to 37.degree. C. and is flowable at temperatures in the range 38.degree. to 52.degree. C. The drug delivery polymer is not limited to the delivery of soluble or liquid drug formulations. For example, the polymer can be used as a matrix for stabilizing and retaining at the site of injection drug-containing microspheres, liposomes or other particulate-bound drugs.

A particularly suitable vehicle for intraocular injection is sterile distilled water in which one of the agonist of VEGFR-1, antagonist of VEGFR-1 and combined preparation VEGFR-1 agonist/second active compound is formulated as a sterile, isotonic solution, properly preserved. Yet another ophthalmic preparation may involve the formulation of such agonist, antagonist or combined preparation with an agent, such as injectable microspheres or liposomes, which provides for slow or sustained release. Other suitable means for the intraocular introduction include implantable drug delivery devices.

The ophthalmic preparations of the present invention, particularly topical preparations, may include other components, for example ophthalmically acceptable preservatives, tonicity agents, cosolvents, wetting agents, complexing agents, buffering agents, antimicrobials, antioxidants and surfactants, as are well known in the art. For example, suitable tonicity enhancing agents include alkali metal halides (preferably sodium or potassium chloride), mannitol, sorbitol and the like. Sufficient tonicity enhancing agent is advantageously added so that the formulation to be instilled into the eye is hypotonic or substantially isotonic. Suitable preservatives include, but are not limited to, benzalkonium chloride, thimerosal, phenethyl alcohol, methylparaben, propylparaben, chlorhexidine, sorbic acid and the like. Hydrogen peroxide may also be used as preservative. Suitable cosolvents include, but are not limited to, glycerin, propylene glycol and polyethylene glycol. Suitable complexing agents include caffeine, polyvinylpyrrolidone, beta -cyclodextrin or hydroxypropyl-beta -cyclodextrin. Suitable surfactants or wetting agents include, but are not limited to, sorbitan esters, polysorbates such as polysorbate 80, tromethamine, lecithin, cholesterol, tyloxapol and the like. The buffers can be conventional buffers such as borate, citrate, phosphate, bicarbonate, or Tris-HCl.

The formulation components are present in concentrations that are acceptable to the extraocular or intraocular site of administration. For example, buffers are used to maintain the composition at physiological pH or at slightly lower pH, typically within a pH range of from about 5 to about 8. Additional formulation components may include materials which provide for the prolonged ocular residence of the extraocularly administered therapeutic agent so as to maximize the topical contact and promote absorption. Suitable materials include polymers or gel forming materials which provide for increased viscosity of the ophthalmic preparation. Chitosan is a particularly suitable material as an ocular release-rate controlling agent in sustained release liquid ophthalmic drug formulations (see U.S. Pat. No. 5,422,116, Yen, et. al.) The suitability of the formulations of the instant invention for controlled release (e.g., sustained and prolonged delivery) of an ophthalmic treating agent in the eye can be determined by various procedures known in the art, e.g., as described in Journal of Controlled Release, 6:367-373, 1987, as well as variations thereof.

Pharmaceutical compositions suitable for use in the invention include compositions wherein the active ingredients are contained in an effective amount to achieve the intended purpose. The determination of an effective dose is well within the capability of those skilled in the art.

For any compound, the therapeutically effective dose can be estimated initially either in cell culture assays, e.g., of neoplastic cells, or in animal models, usually mice, rabbits, dogs, or pigs. The animal model may also be used to determine the appropriate concentration range and route of administration. Such information can then be used to determine useful doses and routes for administration in humans.

Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio between toxic and therapeutic effects is the therapeutic index, and it can be expressed as the ratio, LD50/ED50. Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the ED50 with little or no toxicity. The dosage varies within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active moiety or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week, or once every two weeks depending on half-life and clearance rate of the particular formulation.

Preferably, the medicament is administered so that the molecule of the present invention is given at a dose between 1 µg/kg and 10 mg/kg, more preferably between 10 µg/kg and 5 mg/kg, most preferably between 0.1 and 2 mg/kg. Preferably, it is given as a bolus dose. Continuous infusion may also be used and includes continuous subcutaneous delivery via an osmotic minipump. If so, the medicament may be infused at a dose between 5 and 20 µg/kg/minute, more preferably between 7 and 15 µg/kg/minute. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc. Pharmaceutical formulations suitable for oral administration of proteins are described, e.g., in US 5,008,114; 5,505,962; 5,641,515; 5,681,811; 5,700,486; 5,766,633; 5,792,451; 5,853,748; 5,972,387; 5,976,569; and 6,051,561.

The invention will be fully illustrated by the following figures and examples. All of the starting materials and reagents disclosed below are known to those skilled in the art, and are available commercially or can be prepared using well known techniques.

### BRIEF DESCRIPTION OF THE FIGURES

**Figure 1****: Effect of VEGF on transepithelial electrical resistance (TER) and transepithelial flux of tritiated inulin of cultured ARPE-19 as a function of time.** Confluent ARPE-19 cells grown on transwell polyester filters for 4 weeks form monolayers with constant TER. These monolayers were incubated with various concentrations of VEGF. Paracellular permeability was assessed by TER recording or apical to basal [³H] inulin flux measurements.
   **Figure 1A****:** Time course of TER decrease after incubation with 0.2, 0.5 and 1nM VEGF. Net TER results from the difference between 10 measures of TER cell monolayer and TER filter alone. Results are expressed as percentage of net TER in comparison with net TER before exposure to VEGF. Mean ± SD%TER result from three separate experiments.
   **Figure 1B****:** Effect of 1nM on TER ARPE-19 monolayers measured at 6, 24, 36, 48 and 65 hours.
   **Figure 1C****:** Time course of inulin flux across ARPE-19 monolayers after incubation with 1nM VEGF. ³H-inulin flux was measured by counting radioactivity (indicated by cpm) in 10 µl during a period of 10 to 180 minutes. Data are representative of 3 experiments.
   **Figure 1D****:** Inulin flux across RPE monolayer 10 minutes after incubation of various concentration of VEGF ranging from 0.1 to 1nM VEGF.
**Figure 2****: Effect of 1nM VEGF on occludin distribution as shown by immunohistochemistry on ARPE-19 monolayers at 20 min, 24 hours and 65 hours.. Fluorescence microphotographies.**
   **Figure 2A****:** Control cells showing hexagonal regular distribution representing tight junctions (X25).
   **Figure 2B****:** 20 min after VEGF incubation. Disruption of tight junctions (arrows) and punctate irregular occludin pattern at the membrane (arrowheads) are observed (X25).
   **Figure 2C****:** 24 hours after VEGF incubation. Increased rupture of tight junctions (arrows), occludin distribution from the membrane to the cytoplama and nulcei (stars) c:Inset:X50. Punctate irregular occludin pattern at the membrane (arrowheads).
   **Figure 2D****:** 65 hours after VEGF incubation. Recovery of occludin membrane localization with remaining cytoplasmic occludin (stars) (X25).
**Figure 3****: Effect of hypoxic conditions (10nM CoC12) on VEGF, VEGFR-1 and VEGFR-2 expression and on TER of ARPE-19 monolayers.**
   **Figure 3A****:** VEGF RT-PCR products from ARPE-19 cells, 16 hours after exposure to various concentrations of CoC12. Results are expressed as means ± SD of VEGF/b-actin ratio (n= 3 separate experiments).
   **Figure 3B****:** Time course of VEGFR-1 expression after exposure to 10nM CoC12. Results are expressed as means ± SD of VEGFR-1/b-actin ratio (n= 3 separate experiments).
   **Figure 3C****:** Time course of VEGFR-2 expression after exposure to 10nM CoC12. Results are expressed as means ± SD of VEGFR-2/b-actin ratio (n= 3 separate experiments).
   **Figure 3D****:** Time course of TER monitoring after exposure of ARPE-19 monolayers to 10nM CoC12. Results are expressed as percentage of mean net TER in comparison with mean TER before exposure to CoC12. Means ± SD result from three separate experiments.
**Figure 4****: Effect of PIGF-1, agonist of VEGFR-1 or J-IgG, an agonist of VEGFR-2 on ARPE-19 monolayers permeability**
   **Figure 4A****:** Time course of TER decrease after incubation with 1nM VEGF 165 or 2nM P1GF-1. Net TER results from the difference between 10 measures of TER cell monolayer and TER filter alone. Results are expressed as percentage of net TER in comparison with net TER before exposure to VEGF or P1GF-1. Mean ± SD result from three separate experiments
   **Figure 4B****:** Time course of inulin flux across ARPE-19 monolayers after incubation with 1nM VEGF 165 or 2nM P1GF-1. ³H-inulin flux was measured by counting radioactivity (indicated by cpm) in 10 µl during a period of 10 to 180 minutes. Data are representative of 3 experiments.
   **Figure 4C****:** Time course of inulin flux across ARPE-19 monolayers after incubation with 4nM J-IgG, a specific agonist of VEGFR-2. ³H-inulin flux was measured by counting radioactivity (indicated by cpm) in 10 µl during a period of 10 to 180 minutes. Data are representative of 3 experiments.
**Figure 5****: Effect of antisense oligonucleotides directed at either VEGFR-1 or VEGFR-2 on ARPE-19 monolayers VEGF(1nM)- induced permeability**
   **Figure 5A****:** ARPE-19 cells monolayers were incubated with 1nMVEGF after 24 hours of pre treatment with antisense oligonucleotides directed at VEGFR-1. Net TER results from the difference between 10 measures of TER cell monolayer and TER filter alone. Results are expressed as percentage of mean net TER in comparison with mean TER before exposure to VEGF. Mean ± SD result from three separate experiments
   **Figure 5B****:** ARPE-19 cells monolayers were incubated with 1nM VEGF after 24 hours of pretreatment with antisense oligonucleotides directed at VEGFR-2. Net TER results from the difference between 10 measures of TER cell monolayer and TER filter alone. Results are expressed as percentage of mean net TER in comparison with mean TER before exposure to VEGF. Mean ± SD result from three separate experiments.
   **Figure 5C****:** Down-regulation of VEGFR-1 expression by specific antisense oligonucleotides, three hours after 10 nM CoC12 exposure. No effect of the scrambled oligonucleotides was observed on VEGFR-1 expression.
   **Figure 5D****:** Down-regulation of VEGFR-2 by specific antisense oligonucleotides, there hours after 10nM CoC12 exposure. No effect of the scrambled oligonucleotides was observed on VEGFR-2 expression.
**Figure 6****: Zonula Occludens immunostaining on whole flat-mounted RPE.**
   **Figure 6A****:** 5 hours after intravitreal BSS (balance Saline Solution) injection.
   **Figure 6B and 6C****:** 5 hours after PLGF-1(100 nM) intravitreal injection. Cell junctions are not more observed and a diffuse cytoplasmic distribution of zonula occludens protein is recognized. Spaces can be distinguished in between RPE cells (Arrows).

### EXAMPLE 1:

### 1.1 Materials and methods

### ARPE-19 cell Culture

ARPE-19 (ATCC CRL-2302), a spontaneous immortalized human RPE cell line (Dunn et al., Exp Eye Res. 1996 Feb;62(2):155-169) was cultured in a mixture 1:1 of Dulbecco's modified Eagle's medium (DMEM) and Nutrient Mixture F12, with Hepes buffer (Gibco, Grand Island, NY, U.S.A.) supplemented with 10% fetal bovine serum (FBS). ARPE-19 cells were seeded at a density of 1.5x10⁵ cells/cm² onto either 25cm² flasks (Becton Dickinson, USA) or on transwell polyester filters (0.4µm pore size, 6.5mm in diameter) (Transwell; Costar, Cambridge, MA). Seeding at this cell density on transwells allowed for the formation of monolayers. The cultures were kept at 37°C in 5% CO₂ humidified atmosphere.

### Treatment of ARPE-19 cultures

VEGF165 (J. Plouet J Biol Chem. 1997 May 16;272(20):13390-6) was added at concentrations ranging from 0.15 to 1 nM / culture. To determine which receptor does mediate VEGF-induced permeability, confluent ARPE-19 monolayers grown on transwells were incubated either with 2nM P1GF-1 (agonist for VEGFR-1) (Relia Tech GmbH, Braunschweig, Germany) or with 4nM J-IgG, an agonist of VEGFR-2 (Ortega et al., 1997, Am J Pathol 151:1215-24). For transepithelial electrical resistance (TER) assessments and for inulin flux measurements, modulators were added to serum-free medium. To mimic hypoxic conditions, ARPE-19 monolayers were incubated with CoCl₂ as previously described (Piret et al., Ann N Y Acad Sci. 2002 Nov;973:443-7).

### Transepithelial Electrical Resistance Measurements

Experiments were performed on confluent ARPE19 cells monolayers, grown on transwell filters. Changes in transepithelial resistance (TER) across the monolayer were monitored every week until the 4^{th} week of culture using the resistance system (Millicell; Millipore, Bedford, MA) connected to dual Ag-AgCl electrodes. Net TER values were calculated by subtracting the mean resistance determined for ten plastic filters in absence of cells from the values recorded for each cell monolayer grown on similar filters. Resistance was expressed in Ohms/cm². After TER stabilization (3 to 4 weeks cultures), various concentrations of VEGF 165 or P1GF-1 were added. Changes in TER were monitored after 1,10 and 30 minutes, and 1, 6, 24, 36, 48 and 65 hours of incubation with VEGF, P1GF-1 or medium. Δ(TER) corresponds to the difference between TER values of ARPE 19 monolayers after and before stimulation (control cells). Values are illustrated as the mean of three or more independent cultures.

### Permeability Assay by [³H]Inulin Flux

The paracellular permeability of cultured RPE cells was measured by the passive permeation of [³H]inulin across confluent cells grown on filters. The apical and basal sides of the cell layers were bathed in 300 µl and 600 µl serum-free culture media containing the different stimulants and supplemented with 1mM unlabeled inulin (Sigma France). These incubation volumes were chosen to avoid interference from hydrostatic pressure. [³H]inulin (2 µCi; specific activity, 1.95 mCi/millimole) was added to the medium bathing the apical side of the cells. Aliquots of 10µl were collected from the lower chambers at different time points during 120 minutes. The radioactivity in the collected aliquots was counted in a liquid scintillation analyzer (Packard, Meriden, CT, USA). All measurements were performed in triplicates and illustrated as the mean counts per minute of test culture.

### Immunohistochemistry on ARPE-19 monolayers

Confluent RPE cells grown on polyester filters were fixed with 4% parafolmaldehyde in PBS for 20 minutes at room temperature, rinsed and incubated in PBS containing 15% skimmed milk for 15 minutes to block nonspecific binding sites. Cells were incubated for 60 minutes in a humidified atmosphere at room temperature with a rabbit polyclonal Occludin antibody (Zymed Laboratories, San Francisco, CA) in PBS containing 1% skimmed milk. The cells were then rinsed in PBS containing 1% BSA and further incubated for 60 minutes with anti-rat Texas red (Jackson Immuno Research, Baltimore, PA). Filters were sealed between glass slide and coverslips and examined under a fluorescence microscope (Leica, Switzerland).

### Expression of VEGF, VEGFR-1 and VEGFR-2 using semi-quantitative RT-PCR

For RT-PCR analysis in normal and hypoxic conditions, cells were grown on 25cm² flasks. Total mRNA was extracted from each cell culture according to the manufacture's instruction (Qiagen, Valencia, CA). The extracted RNA was quantified, and 2 µg of each sample were used to make cDNA using SuperScript 11 (Invitrogen, Life Technologies). RT-PCR quantification was carried out in a linear range. The PCR conditions consisted of denaturation at 95°C for 30 seconds, annealing at 55°C (β-action), 57°C (VEGFR-1, 2 and VEGF) for 30 seconds and extension at 72°C for 60 seconds. The reaction was initiated by adding two units of Taq DNA polymerase (Invitrogen), followed by 35 cycles for VEGF, VEGFR-1, 2 and 25 cycles for (β-actin. The primers used in this experiment were SEQ ID N°4: 5'-CCTCCGAAACCATGAACTTT-3' (forward), SEQ ID N°5: 5'-AGAGATCTGGTTCCCGAAAC-3' (reverse) for VEGF (637bp) (Nakayama et al., 2002, Cancer Lett 176:215-223), SEQ ID N°6: 5'-CAAGTGGCCAGAGGCATGGAGTT-3' (forward), SEQ ID N°7: 5'-GATGTAGTCTTTACCATCCTGTTG-3' (reverse) for VEGFR-1 (498bp) (Masood et al., 1997, Proc Natl Acad Sci U S A 94:979-984.), SEQ ID N°8: 5'-GAGGGCCTCTCATGGTGATTGT-3' (forward), SEQ ID N°9: 5'-TGCCAGCAGTCCAGCATGGTCTG-3' (reverse) for VEGFR-2 (706bp) (Masood et al., 1997, Proc Natl Acad Sci U S A 94:979-984.), SEQ N°10: 5'-AGGAGAAGCTGTGCTACGTC-3' (forward) and SEQ N°11: 5'-AGGGGCCGGACTCGTCATAC-3' (reverse) for (β-actin (465bp).

### Down-regulation of VEGFR-1 and VEGFR-2 using antisense oligonucleotides

A preliminary experiment was performed to confirm the specificity and efficacy of antisense oligonucleotides (ODN) directed at either VEGFR-1 or VEGFR-2. For this experiment, and in order to have enough material for RNA extraction, cells were seeded on 25cm² flasks. After 7 days of culture, cells were exposed to CoC12 (hypoxic conditions) with or without pre treatment with specific antisense ODN. The following phosphorothioate ODN were used: VEGFR-1 antisense: SEQ ID N°1: 5'-GTAGCTGACCATGGTGAGCG-3', VEGFR-1 scrambled: SEQ ID N°12: 5'-ATCAGCGTGTGAGCACGGTG-3', VEGFR-2 antisense: SEQ ID N°13: 5'-GCATCTCCTTTTCTGAC-3' and VEGFR-2 scrambled: SEQ ID N°14: 5'-TTAATCGTTCTCTGCCC-3'.

Oligonucleotides (8.0 µg) and 20 µl of Lipofectamine 2000 reagent (Invitrogen) were diluted separately each in 500 µl of OptiMEM I Reduced Serum Medium and incubated at room temperature for 5 min. Oligonucleotides and Lipofectamine 2000 were then mixed and incubated together for another 20 min. This mixture was added to each flask and another 2 ml Opti MEM was added to each flask to prevent from dryness, and then cells were incubated at 37°C for 5 hours. The transfection mixture was then gently removed and replaced with DMEM containing 10% FBS. 24 hours later 10 µM CoC12 was added for three hours. The cells were then collected for RNA extraction and VEGF receptors expression analysis using RT-PCR as previously described.

Antisense oligonulceotides were then used to pre treat confluent ARPE-19 monolayers before TER measurements under VEGF stimulation. Oligonucleotides (0.8 µg) and 2.0 µl of Lipofectamine 2000 reagent (Invitrogen) were diluted separately, each in 50 ul of OptiMEM I Reduced Serum at room temperature for 5 min. Oligonucleotides and Lipofectamine 2000 were then mixed and incubated together for another 20 min. This mixture was then added to the cells in 200 µl Opti MEM and incubated at 37°C for 5 hours. After 5 hours, The transfection mixture was gently removed and replaced by DMEM 2% FBS for 24 h before TER monitoring under 1nM VEGF stimulation as previously described.

### Effect of intravitreous injection of PIGF-1 on RPE cells junctions using ZO-1 immunohistochemistry on flat-mounted RPE/ choroid/ sclera.

To confirm the effect of P1GF-1 on RPE cells junctions in vivo, female Lewis rats, 6-7 weeks old and weighing 150-200 g (IFFA CREDO, Lyon, France) were used. Experiments were conducted in accordance with the ARVO Statement for the Use of Animals in Ophthalmic and Vision Research. Rats were held in the vivarium for 1 week before inclusion in the study.

For the experimentation, rats were anesthetized by intraperitoneal pentobarbital injection (40 mg/kg). They received a single intravitreous injection of 10ng/ml (n=4) P1GF-1 diluted in 5µl of PBS or 5µl of PBS alone (n=2). Other control rats (n=2) did not receive any injection. Five hours after P1GF-1 injection, rats were sacrificed by an overdose of pentobarbital, and rat RPE tight junctions were labeled by immunolocalization of the tight junction-associated protein zonula occludens-1 (ZO-1). Eyes were enucleated, fixed in 2% paraformaldehyde for 1 hour. Biomicroscopic dissection of the sclera, choroid and RPE complex was achieved after removal of the cornea and extirpation of the lens, vitreous body and retina. The sclera, choroid and RPE complex was washed three times for 5 minutes in 1% bovine serum albumin/phosphate-buffered saline (BSA/PBS) then washed three times for 10 minutes in 1%BSA/PBS and 0.2% saponin and subsequently incubated overnight at 4 °C with rat anti-ZO-1 antibody (1:200, Chemicon, Temecula, CA) diluted in 1%BSA/PBS and 0.2% saponin. ZO-1 was visualized by incubation with Alexa-conjugated goat anti-rat IgG (1:100, Molecular Probes, Eugene, Oregon, USA). The complex was finally mounted with great care to prevent folding using glycerol and observed using appropriate filters using a confocal microscope (Zeiss LSM 510, Switzerland).

### Statistical Analysis

All experiments were repeated at least three times. The results are expressed as mean ± standard deviation. Statistical analysis was performed using the two-tailed Student's *t* test, P<0.05 was considered significant.

### 1.2 Results

### Effect of VEGF on RPE tight-junctions and permeability

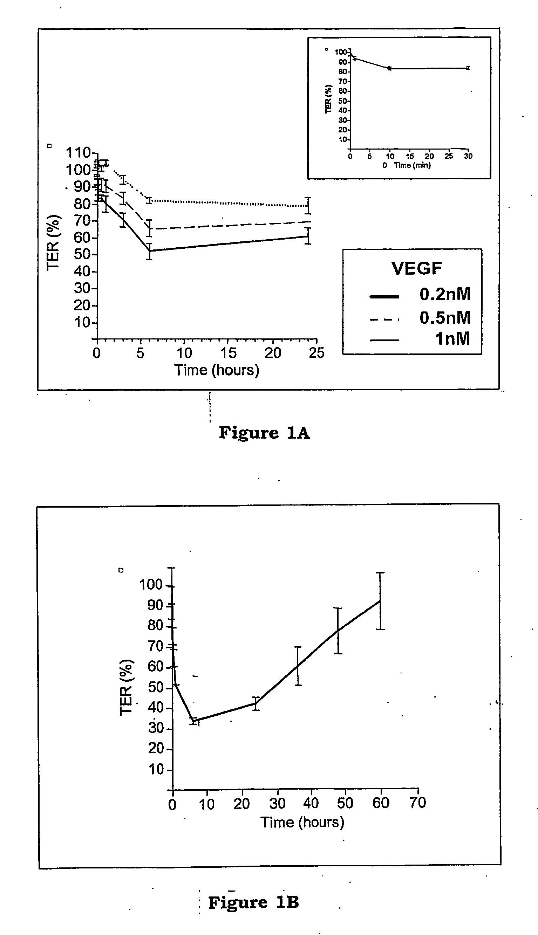

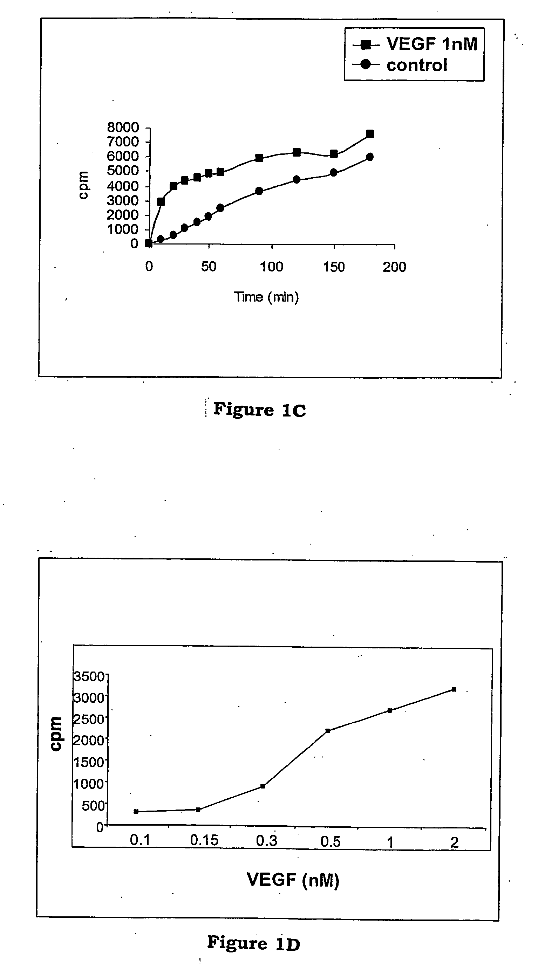

After 4 weeks of culture on transwells, ARPE-19 cells have formed confluent monolayers with a constant mean TER level of 60±8.6 Ohm/cm². Addition of 0.2nM to 1nM VEGF induces a dose-response decrease of RPE TER (Fig. 1A and 1D). The TER decrease is observed as early as 10 min after exposure to 1nM VEGF (Fig. 1A, inset), accentuating during the first 6 hours, and then slowly returning to normal values in about 65 hours (Fig. 1B). The decrease of TER in cells being suggestive of an alteration of paracellular permeability, flux of inulin, a well-known paracellular tracer was studied. This inert hydrophilic molecule is a small enough extracellular probe to permeate the tight junctions network in RPE cells. Inulin flux through RPE monolayers was recorded as a function of time over 180 min (Fig. 1C). Treatment of RPE cells with VEGF (1nM) increases the radioactivity measured in the basal side of the well, when compared to non-treated cells (P<0.05). The inulin flux increase across stimulated RPE monolayers was maximal as early as 10 min, lasted for 60 min, and then slowly faded.

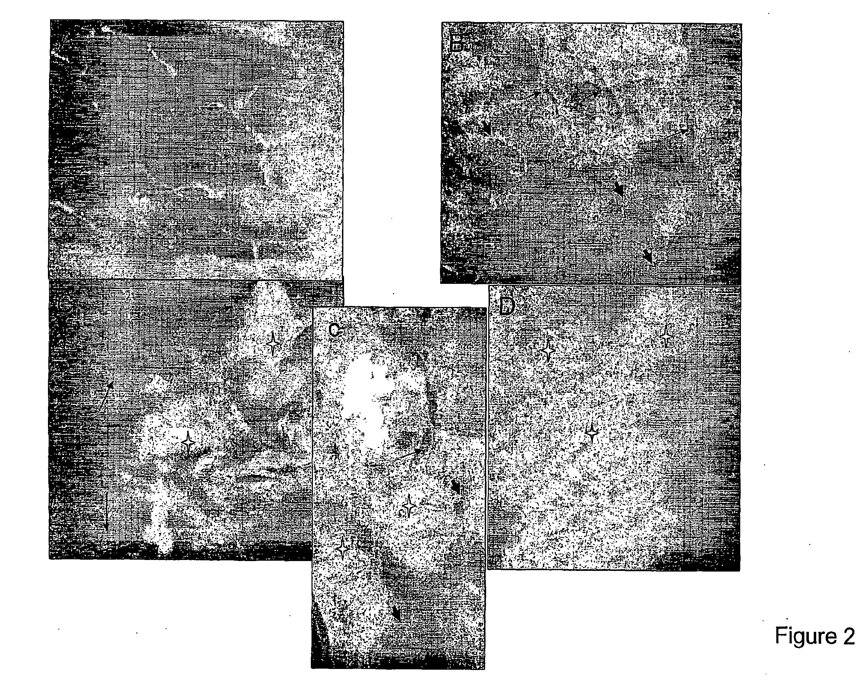

The TER measurements and [³H] inulin fluxes strongly suggest that VEGF may affect the paracellular permeability of RPE tight-junctions. Because changes in tight-junction structures may be conterpart of functional modulations, VEGF-stimulated ARPE-19 monolayers were examined to determine alteration of tight-junction associated proteins. After 4 weeks of culture untreated ARPE-19 monolayers shows continuous occludin labeling around the cell periphery (Figure 2A). As early as 20 minutes after VEGF addition, and more markedly after 24 hours, discontinuous occludin labeling is observed at the periphery of the cells, spaces between cells can be observed showing unsealed junctions (Fig. 2B and C, arrows). A change in occludin distribution is observed with labeling in the cytoplasm and the nuclei of cells associated to reduced labeling at the cell membrane (Fig. 2B and C, stars). Irregular punctate occludin pattern can be observed at high magnification at the membrane of some cells (Fig. 2B and C, arrowheads). At 65 hours after VEGF addition (1nM), the initial regular distribution of occludin in the tight junction is reforming with some remaining occludin localization in the cytoplasm (Fig. 2D).

### Effect of hypoxic conditions on VEGF and VEGF receptors expression, and RPE Permeability

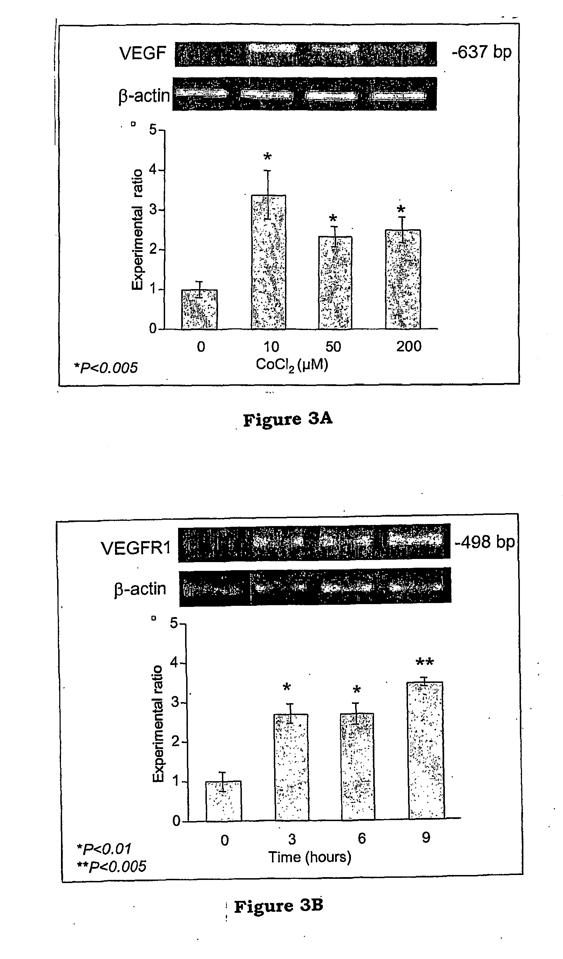

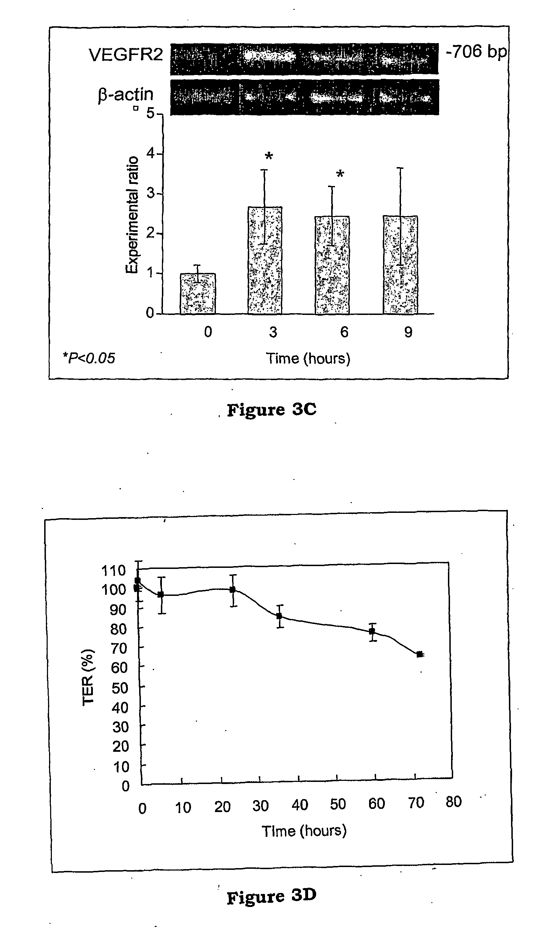

VEGF expression is up-regulated by hypoxia, as confirmed by exposure of ARPE-19 cells to Cocl2. Indeed, VEGF expression is significantly up-regulated sixteen hours after CoC12 exposure (mimicking hypoxic conditions) (10, 50 and 200µM) (p<0.005 for all doses) (Fig. 3A). The inventors therefore have examined the influence of hypoxic conditions of VEGF receptors expression and RPE monolayers permeability. VEGFR-1 and VEGFR-2 are expressed in RPE cells in culture under basal conditions (Fig. 3A and 3B) and in cells with tight junctions (not shown). Under hypoxic conditions VEGFR-1 expression is already up-regulated at 3 and 6 hours (p<0.01) with increased expression 9 hours after exposure (p<0.005). VEGFR-2 expression is also up-regulated under these conditions at 3 and 6 hours (p<0.05), but decreasing thereafter (Fig 3C). Since hypoxia induces an up-regulation of VEGF receptors, the inventors have followed the changes in ARP-19 TER as a function of time after exposure to hypoxic conditions (10µM CoCl2). Exposure of cells to 10µM CoCl2 induces a delayed but prolonged decrease of TER, beginning at 24 hours after exposure and lasting for at least 3 days (Fig 3D) suggesting that endogenously produced VEGF may modulate RPE cells permeability in vitro.

### Effect of specific agonists of VEGF receptors or antisense oligonucleotides directed at VEGF receptors on VEGF-induced RPE paracellular permeability.

In order to determine which VEGF receptor, VEGFR-1 or VEGFR-2 is mediating the VEGF-induced paracellular permeability, the inventors have used two different strategies. They examined the effect on TER RPE monolayers of P1GF-1, agonist of VEGFR-1 and of J-IgGI, an agonist of VEGFR-2 (Ortega et al., 1997, Am J Pathol 151:1215-24). In a separate experiment, they examined the potentially inhibitory effect on VEGF-induced TER decrease, of RPE pre treatment with antisense oligonucleotides directing at either VEGFR-1 or VEGFR-2.

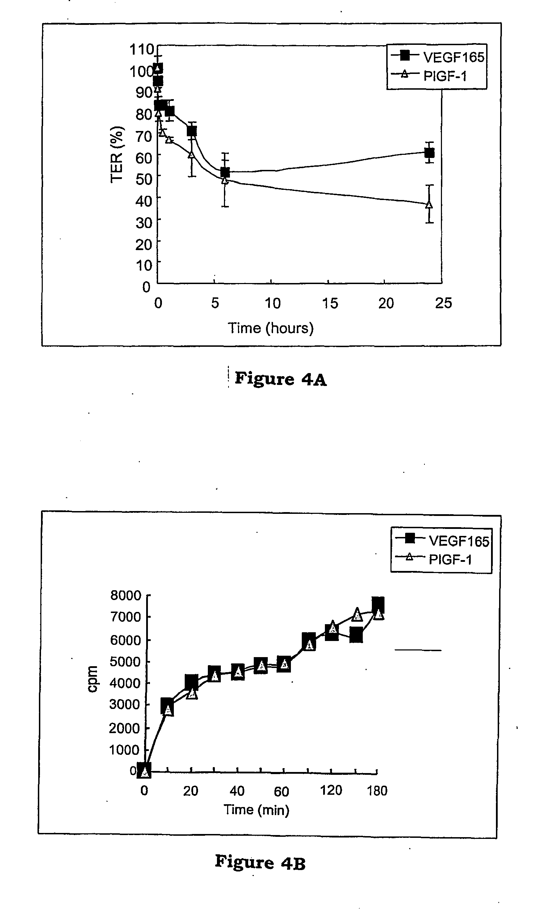

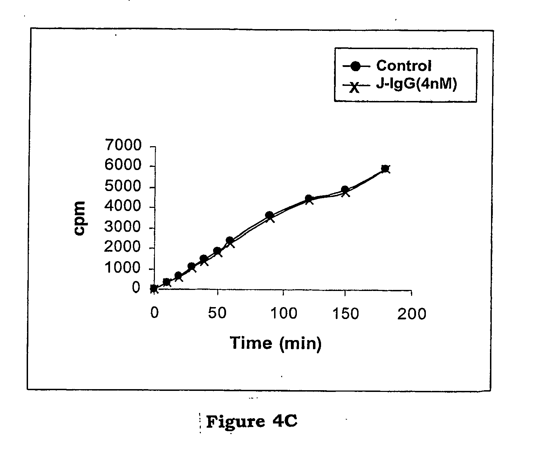

P1GF-1 (2nM) induces a decrease in RPE TER monolayer, similar in kinetics and intensity to the one induced by VEGF 1nM (Fig 4A and Fig 4B). On the other hand, J-IgG I, an agonist of VEGFR-2 does not induce any decrease of TER (not shown), and does not increase the flux of [3H] inulin across RPE monolayers demonstrating that activation of VEGFR-2 does not seem to influence paracellular permeability (Fig.4C).

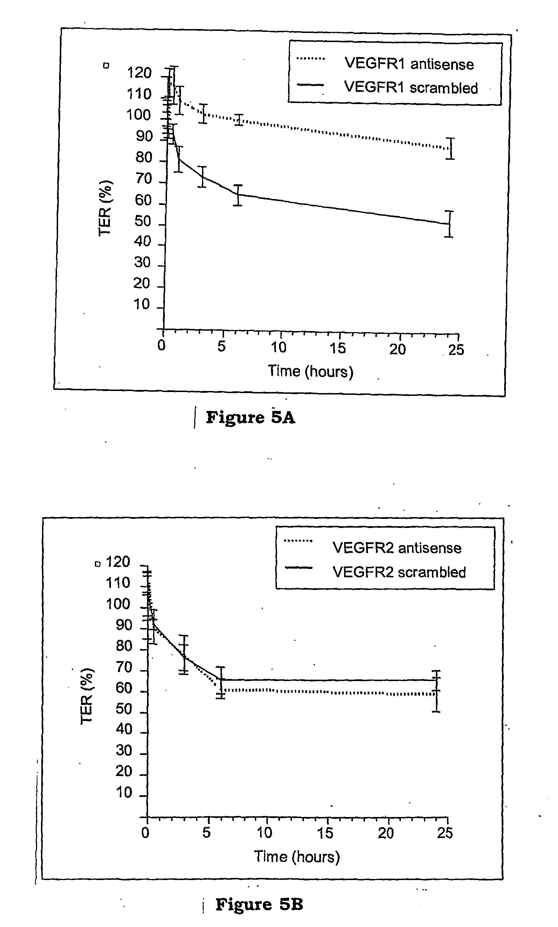

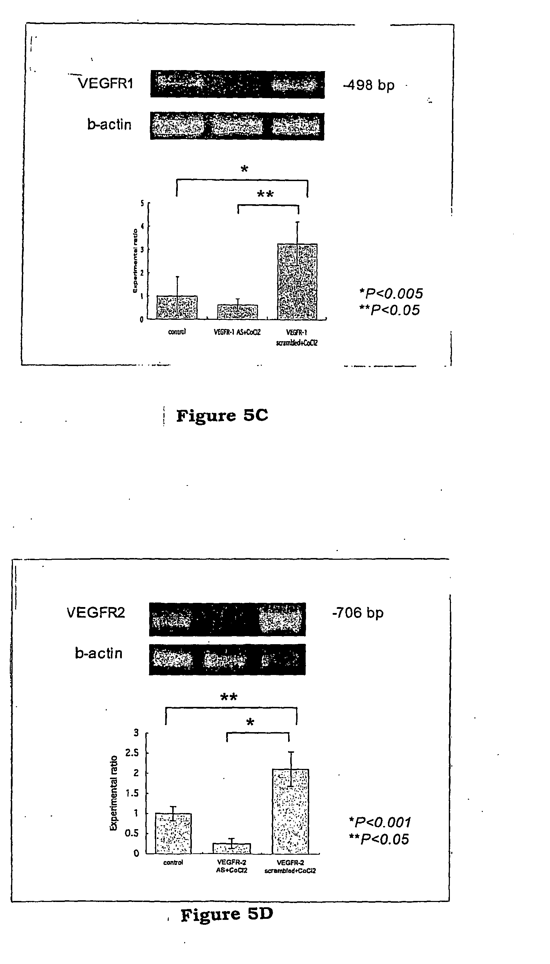

On the other hand, twenty-four hours of RPE cells pretreatment with an antisense oligonucleotide directed at VEGFR-1 abolishes the effect of VEGF (1nM) on TER RPE monolayers. No inhibitory effect is observed when the scrambled oligonucleotide is used (Fig 5A). Pretreatment of RPE cells with an antisense directed at VEGFR-2 does not influence the effect of VEGF on RPE cells TER (Fig 5B). The efficacy and specificity of these oligonulceotides was confirmed by the specific down regulation of VEGF receptors under hypoxic conditions. The antisense oligonucleotides directed at either VEGFR-1 or VEGFR-2 significantly downregulated the expression of the receptors, three hours after exposure to 10 µM CoC12. Scrambled oligonucleotides failed to impede the expression of either of the VEGF receptors (Fig 5C and 5D).

### Effect of P1GF-1 on rat RPE tight junctions in vivo

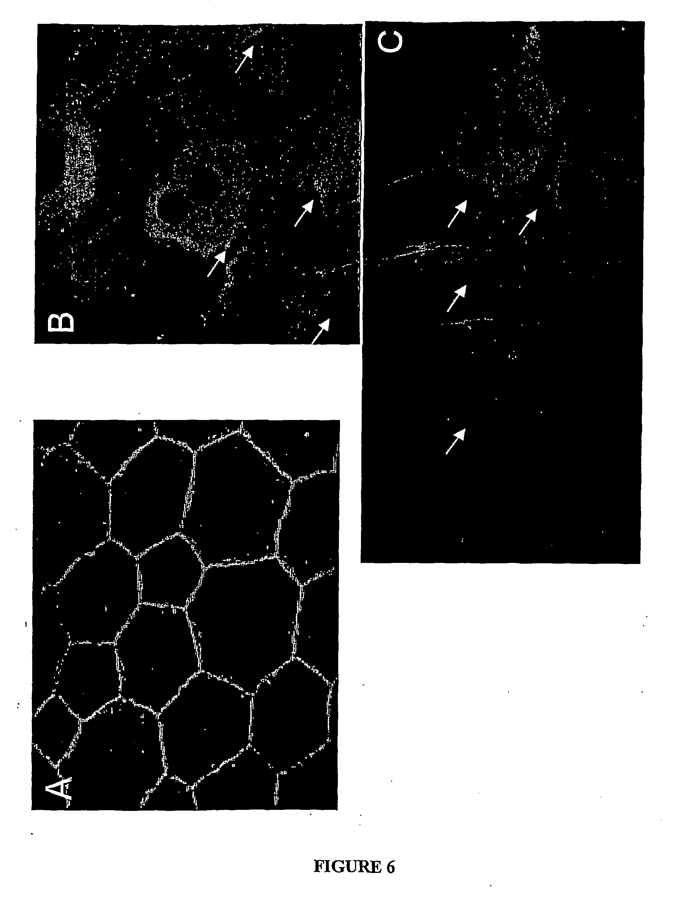

In order to confirm the potential effect of VEGF through VEGFR-1 on RPE cells paracellular permeability in vivo, the inventors have injected P1GF-1 in the vitreous of rats and have looked for any changes in tight-junction associated protein distribution by immunolocalization on RPE flat-mounts 5 hours later (according to the in vitro kinetics). ZO-1 immunohistochemistry on whole flat-mounted RPE/ choroids/ sclera shows regular localization of all around the cell membranes in control rats RPE (not shown) and in PBS-injected rats (Fig. 6A). A very different pattern of ZO-1 distribution was observed on RPE cells of rats at 5 hours after P1GF-1 (100ng/ml, 2nM) intravitreous injection. In the later, spaces between cells and irregular punctate ZO-1pattern is observed associated to a cytoplasmic distribution of the tight-junction associated protein (Fig. 6B).

### 1.3 Discussion

The results show that VEGF modulates tight-junctions of human RPE cells in vitro. The expression of VEGF and its receptors is up-regulated under hypoxic conditions. However, while VEGFR-1 expression is sustained, expression of VEGFR-2 is transitory. These findings suggest that VEGF, produced in retinal diseases in response to hypoxia may influence not only the internal hemato retinal barrier (retinal endothelial cells), but also the external hemato retinal barrier, contributing to the pathogenesis of macular edema and maybe to the growth of neo vessels from the choricapillaries through the RPE.

P1GF-1 which specifically binds to VEGFR-1 (Christinger et al., 2004, J Biol Chem 279:10382-10388), mimics VEGF-induced TER decrease in ARPE-19 in vitro, and induces a structural changes of tight-junctions in the rat eye in vivo. This suggests that the effect of VEGF on RPE permeability could be mediated mostly by Flt-1 and that it does occur in vivo. The inventors have found that J-IgG, a specific VEGFR-2 agonist, had no effect on inulin flux, reinforcing the hypothesis that VEGF-induced paracellular permeability on RPE cells is not mediated by VEGFR-2 but rather by VEGFR-1.

The present result demonstrates that VEGF may regulate the integrity of the external hemato-retinal barrier by a direct effect on tight junctions, and that this regulation is mostly mediated by VEGFR-1. The facts that hypoxic conditions increases permeability of RPE cells in vitro and that P1GF-1 is able to open tight junctions of the rat RPE in vivo are in favor of a real implication of this mechanism in the pathogenesis of retinal diseases associated to VEGF up regulation.

## Claims

1. An antagonist of vascular endothelial growth factor receptor 1 (VEGFR-I) for use in the prevention or the treatment of a subject suffering from an ocular disease wherein disorders of the retinal pigment epithelium (RPE) permeation are involved, wherein the antagonist is an antisense oligonucleotide of the mRNA encoding VEGFR-I, said antisense oligonucleotide having the sequence SEQ ID N°1, and wherein the ocular disease is selected from the group comprising macular edema, central serous chorioretinopathy, epitheliopathy and age related macular degeneration (ARMD).

2. A pharmaceutical composition comprising an antisense oligonucleotide of the mRNA encoding VEGFR-I, said antisense oligonucleotide having the sequence SEQ ID N°1, together with a pharmaceutically acceptable carrier.

3. The pharmaceutical composition according to claim **2,** for preventing or treating a subject suffering from an ocular disease wherein disorders of the RPE permeation are involved, the ocular disease being selected from the group comprising macular edema, central serous chorioretinopathy, epitheliopathy and age related macular degeneration (ARMD).

4. The pharmaceutical composition according to claim **2** or **3** containing a first compound which is an antagonist of VEGFR-I having the sequence SEQ ID N° 1 and a second active compound selected from the group comprising anti angiogenic compounds, non steroidal anti inflammatory, cortico and mineralo corticoids, anti-oxidants, lipids, neurotrophic factors such as ciliary neurotrophic factor or CNTF, glial cell line-derived neurotrophic factor or GDNF, brain-derived neurotrophic factor or BDNF, fibroblast growth factor or FGF, COX inhibitors and antibiotics, as a combined preparation for simultaneous, separate or sequential treatment of an ocular disease.

5. The pharmaceutical composition according to claim **4,** wherein the ocular disease is selected from the group comprising macular edema, central serous chorioretinopathy, epitheliopathy, infections, intraocular inflammations, retinal dystrophy, diabetic retinopathy, ischemic retinopathy, optic neuritis and ocular diseases associated to neovascular and/or fibroglial proliferations.

## Patentansprüche

1. Antagonist des vaskulären endothelialen Wachstumsfaktor-Rezeptor-1 (VEGFR-I) zur Verwendung in der Vorbeugung oder der Behandlung eines Patienten leidend an einer Augenerkrankung, wobei Erkrankungen der retinalen Pigmentepithel-(RPE)-Permeation involviert sind, wobei der Antagonist ein Antisinn-Oligonukleotid der mRNA kodierend für VEGFR-I ist, wobei das Antisinn-Oligonukleotid die Sequenz SEQ ID NO: 1 hat, und wobei die Augenerkrankung ausgewählt ist aus der Gruppe umfassend Makulaödem, zentrale seröse Chorioretinopathie, Epitheliophathie und altersabhängige Makuladegeneration (ARMD).

2. Pharmazeutische Zusammensetzung umfassend ein Antisinn-Oligonukleotid der mRNA kodierend für VEGFR-I, wobei das Antisinn-Oligonukleotid die Sequenz SEQ ID NO: 1 hat, zusammen mit einem pharmazeutisch akzeptablen Träger.

3. Pharmazeutische Zusammensetzung gemäß Anspruch 2 zum Vorbeugen oder zum Behandeln eines Patienten leidend an einer Augenerkrankung, wobei Erkrankungen der RPE-Permeation involviert sind, wobei die Augenerkrankung ausgewählt ist aus der Gruppe umfassend Makulaödem, zentrale seröse Chorioretinopathie, Epitheliophathie und altersabhängige Makuladegeneration (ARMD).

4. Pharmazeutische Zusammensetzung gemäß Anspruch 2 oder 3, enthaltend eine erste Verbindung, die ein Antagonist von VEGFR-I mit der Sequenz SEQ ID NO: 1 ist, und eine zweite aktive Verbindung ausgewählt aus der Gruppe umfassend anti-angiogenetische Verbindungen, nicht-steroidale antiinflammatorische, Kortiko- und Mineralokortikoide, Antioxidantien, Lipide, neurotrophische Faktoren wie Ziliar-neurotrophischer Faktor oder CNTF, Gliazelllinien-abgeleiteter neurotrophischerFaktor oder GDNF, Gehirnabgeleiteter neurotrophischer Faktor oder BDFN, Fibroblasten-Wachstumsfaktor oder FGF, COX-Inhibitoren und Antibiotika, wie eine kombinierte Zusammensetzung zur gleichzeitigen, separaten oder sequentiellen Behandlung einer Augenerkrankung.

5. Pharmazeutische Zusammensetzung gemäß Anspruch 4, wobei die Augenerkrankung ausgewählt ist aus der Gruppe umfassend Makulaödem, zentrale seröse Chorioretinopathie, Epitheliopathie, Infektionen, intraokulare Entzündungen, retinale Dystrophie, diabetische Retinopathie, ischämische Retinopathie, optische Neuritis und Augenerkrankungen assoziiert mit neovaskulären und/oder fibroglialen Proliferationen.

## Revendications

1. Antagoniste du récepteur 1 au facteur de croissance endothélial vasculaire VEGFR-1 pour son utilisation dans la prévention ou le traitement d'un sujet souffrant d'une maladie oculaire, dans laquelle sont impliqués des troubles de la perméation de l'épithélium pigmentaire rétinien (EPR), dans laquelle l'antagoniste est un oligonucléotide antisens du messager ARN encodant VEGFR-1, ledit oligonucléotide antisens ayant une séquence SEQ ID NO. 1, et dans laquelle la maladie oculaire est choisie parmi le groupe comprenant l'oedème maculaire, la choriorétinopathie séreuse centrale (CRSC), l'épithéliopathie et la dégénérescence maculaire liée à l'âge (DMLA).

2. Composition pharmaceutique comprenant un oligonucléotide antisens de l'ARN messager codant pour VEGFR-1, ledit oligonucléotide antisens ayant une séquence SEQ ID NO. 1, en association avec un excipient pharmaceutiquement acceptable.

3. Composition pharmaceutique selon la revendication 2 pour prévenir ou traiter un sujet souffrant d'une maladie oculaire impliquant des troubles de la perméation EPR, la maladie oculaire était choisie dans le groupe comprenant l'oedème maculaire, la choriorétinopathie séreuse centrale (CRSC), l'épithéliopathie et la dégénérescence maculaire liée à l'âge (DMLA).

4. Composition pharmaceutique selon les revendications 2 ou 3 comprenant un premier composé qui est un antagoniste du VEGFR-1 ayant une séquence SEQ ID NO. 1 et un second composé actif choisi dans le groupe comprenant les composés anti-angiogéniques, les anti-inflammatoires non-stéroïdiens, les cortico-corticoïdes et les minéralocorticoïdes, les anti-oxydants, les lipides, les facteurs neurotrophiques tels que le facteur neurotrophique ciliaire ou FNTC, le facteur neurotrophique dérivé d'une lignée des cellules gliales ou GDNF, facteur neurotrophique dérivé du cerveau ou BDNF, facteur de croissance du fibroblaste ou FGF, les inhibiteurs de COX et les antibiotiques, combinés dans une préparation pour un traitement simultané, séparé ou séquentiel d'une maladie oculaire.

5. Composition pharmaceutique selon la revendication 4, dans laquelle la maladie oculaire est choisie dans le groupe comprenant l'oedème maculaire, la choriorétinopathie séreuse centrale (CRSC), l'épithéliopathie, les infections, les inflammations intraoculaires, la dystrophie rétinienne, la rétinopathie diabétique, la rétinopathie ischémique, la névrite optique et les maladies oculaires associées à la prolifération néovasculaire et/ou à la prolifération fibrogliale.


### SEQUENCE LISTING

<110> Novagali
   Octoplus
   Noriko Miyamoto
   Francine Behar-Cohen
   Jean Plouet
   Nadia Normand
<120> Modulating retinal pigmented epithelium permeation by inhibiting or activating VEGFR-1
<130> D 22 792
<160> 14
<170> PatentIn version 3.3
<210> 1
   <211> 20
   <212> DNA
   <213> artificial sequence
<220>
   <223> Antisense oligonucleotide.
<400> 1
   gtagctgacc atggtgagcg 20
<210> 2
   <211> 18
   <212> DNA
   <213> artificial sequence
<220>
   <223> Antisense oligonucleotide.
<400> 2
   aagcagacac cagagcag 18
<210> 3
   <211> 18
   <212> DNA
   <213> artificial sequence
<220>
   <223> Antisense oligonucleotide.
<400> 3
   ccctgagcca tatcctgt 18
<210> 4
   <211> 20
   <212> DNA
   <213> artificial sequence
<220>
   <223> Oligonucleotide primer.
<400> 4
   cctccgaaac catgaacttt 20
<210> 5
   <211> 20
   <212> DNA
   <213> artificial sequence
<220>
   <223> Oligonucleotide primer.
<400> 5
   agagatctgg ttcccgaaac 20
<210> 6
   <211> 23
   <212> DNA
   <213> artificial sequence
<220>
   <223> Oligonucleotide primer.
<400> 6
   caagtggcca gaggcatgga gtt 23
<210> 7
   <211> 24
   <212> DNA
   <213> artificial sequence
<220>
   <223> Oligonucleotide primer.
<400> 7
   gatgtagtct ttaccatcct gttg 24
<210> 8
   <211> 22
   <212> DNA
   <213> artificial sequence
<220>
   <223> Oligonucleotide primer.
<400> 8
   gagggcctct catggtgatt gt 22
<210> 9
   <211> 23
   <212> DNA
   <213> artificial sequence
<220>
   <223> Oligonucleotide primer.
<400> 9
   tgccagcagt ccagcatggt ctg 23
<210> 10
   <211> 20
   <212> DNA
   <213> artificial sequence
<220>
   <223> Oligonucleotide primer.
<400> 10
   aggagaagct gtgctacgtc 20
<210> 11
   <211> 20
   <212> DNA
   <213> artificial sequence
<220>
   <223> Oligonucleotide primer.
<400> 11
   aggggccgga ctcgtcatac 20
<210> 12
   <211> 20
   <212> DNA
   <213> artificial sequence
<220>
   <223> Scrambled oligonucleotide.
<400> 12
   atcagcgtgt gagcacggtg 20
<210> 13
   <211> 17
   <212> DNA
   <213> artificial sequence
<220>
   <223> Antisense oligonucleotide.
<400> 13
   gcatctcctt ttctgac 17
<210> 14
   <211> 17
   <212> DNA
   <213> artificial sequence
<220>
   <223> Scrambled oligonucleotide.
<400> 14
   ttaatcgttc tctgccc 17